# EUROPEAN PATENT SPECIFICATION

(11) **EP 3 223 911 B1**
(45) Date of publication and mention of the grant of the patent: **11.08.2021**
(21) Application number: 14821470.3
(22) Date of filing: 19.12.2014
(51) Int. Cl.: A61Q 11/00, A61K 8/22, A61K 8/85, A61K 8/02, A61K 8/11

(54) **STABLE WHITENING DENTIFRICE COMPOSITION WITH SUPERIOR AESTHETICS**
STABILE BLEICHENDE ZAHNPASTAZUSAMMENSETZUNG MIT ÜBERLEGENER ÄSTHETIK
COMPOSITION DENTIFRICE BLANCHISSANTE STABLE OFFRANT D'EXCELLENTES PROPRIÉTÉS ESTHÉTIQUES

(43) Date of publication of application: 04.10.2017
(73) Proprietor: Colgate-Palmolive Company, New York, NY 10022 (US)
(72) Inventor: FEI, Lin, Kendall Park, New Jersey 08831 (US); CHOPRA, Suman, Monroe, New Jersey 08831 (US); MANDADI, Prakasarao, Flemington, New Jersey 08822 (US)
(74) Representative: Wibbelmann, Jobst
(86) International application number: PCT/US2014/071414
(87) International publication number: WO 2016/099540

(56) References cited:
- WO-A1-98/51270
- WO-A1-2009/023393
- DE-A1-102013 209 892
- US-A- 4 348 378
- US-A1- 2010 247 457
- "Technical Data Sheet Ecocolors. MICRO POWDERS, INC.", , 14 March 2014 (2014-03-14), XP055206014, Retrieved from the Internet: URL:http://www.cosmesi.it/Portals/7/Docume nti/NewsletterSpeciale_In-cosmetics2014/Ec ocolors_TDS.pdf [retrieved on 2015-08-03]
- Stacey Price: "Does Baking Soda Whiten Teeth?", , 4 August 2015 (2015-08-04), XP055206252, Retrieved from the Internet: URL:http://www.colgate.com/en/us/oc/oral-h ealth/cosmetic-dentistry/teeth-whitening/a rticle/sw-281474979043022 [retrieved on 2015-08-04]
- None

## Description

### BACKGROUND

Consumer studies have found that a white color is not necessarily the most favorable color for whitening dentifrices, such as toothpastes. Studies have shown that consumers prefer the aesthetics of blue toothpastes. Moreover, consumers perceive blue toothpastes as having superior whitening efficacy in comparison to white toothpastes. Nevertheless, dentifrice formulations comprising whitening agents, such as peroxide, are highly reactive with colorants such as dyes or lakes, and, consequently, formulating a blue or other colored dentifrice has proven difficult. Accordingly, there remains a need in the art to provide stable, varied colored whitening dentifrice compositions, such as toothpastes, to meet consumer demand.

WO 2009/023393 discloses a composition for minimizing color fading in an oral care composition comprising: a) an organic dye-encapsulated silica shell nanoparticle matrix, b) a physiologically stable fluoride ion-providing compound, c) a physiologically stable peroxide species-providing compound (PSPC) in an amount about 1 % to about 20% by weight.

### BRIEF SUMMARY

The present invention is directed to a stable whitening dentifrice composition including: an orally acceptable vehicle including a whitening agent, and a colorant encapsulated by a protective barrier as defined in the claims, wherein the whitening agent is selected from the group consisting of an oxidizing agent, a reducing agent and a combination thereof, wherein the protective barrier suppresses migration of the colorant into the orally acceptable vehicle for a duration of time of at least about 1 day, and wherein the orally acceptable vehicle is anhydrous.

The present disclosure also provides a method of preparing a whitening dentifrice composition including: (a) mixing a whitening agent into an orally acceptable vehicle to form a mixture; (b) dispersing a colorant encapsulated in a protective barrier into the mixture formed in (a) to form a stable whitening dentifrice composition.

In addition, the present disclosure also provides a method of whitening a tooth surface, the method including: contacting a tooth surface with a stable whitening dentifrice composition including: an orally acceptable vehicle including a whitening agent, and a colorant encapsulated in a protective barrier, wherein the protective barrier suppresses migration of the colorant into the vehicle for a duration of time of at least about one day. Further areas of applicability of the present invention will become apparent from the detailed description provided hereinafter. It should be understood that the specific examples, while indicating typical embodiments, are intended for purposes of illustration only and are not intended to limit the scope of the disclosure.

### BRIEF DESCRIPTION OF THE DRAWINGS

The application file contains at least one drawing executed in color. Copies of this patent application publication with color drawings will be provided by the Office upon request and payment of the necessary fee.

Embodiments of the present disclosure will become more fully understood from the detailed description and the accompanying drawings, wherein:

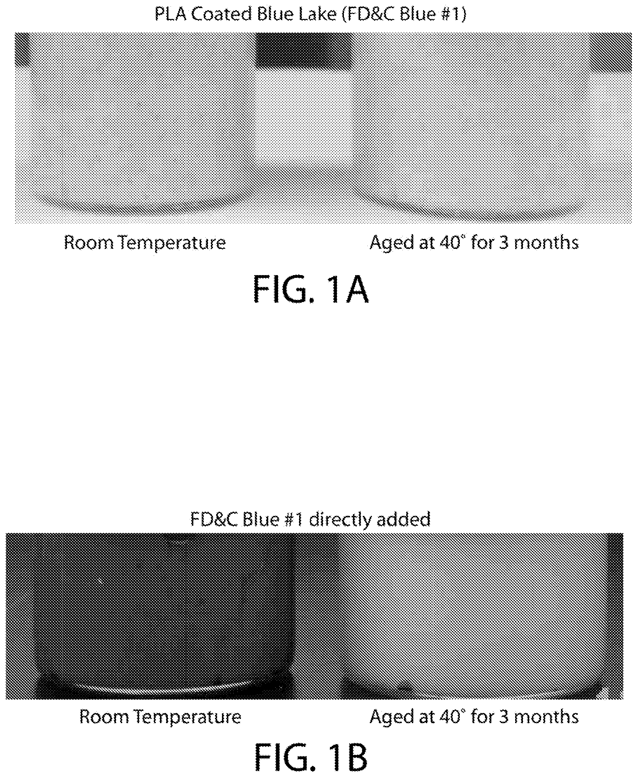

FIG. 1A depicts an embodiment of a stable whitening dentifrice composition prepared as described in Example 1 with a colorant encapsulated with a protective barrier, shortly after preparation at room temperature, and after 3 months at 40°C.
FIG. 1B depicts an embodiment of a whitening dentifrice composition prepared according to Comparative Example 1, using a colorant that is not encapsulated shortly after preparation at room temperature and after 3 months at 40°C.

### DETAILED DESCRIPTION

As used throughout, ranges are used as shorthand for describing each and every value that is within the range. Any value within the range can be selected as the terminus of the range.

Unless otherwise specified, all percentages and amounts expressed herein and elsewhere in the specification should be understood to refer to percentages by weight. The amounts given are based on the active weight of the material.

### Compositions

The present invention provides a stable whitening dentifrice composition, including a whitening agent within an orally acceptable anhydrous vehicle and a colorant encapsulated in a protective barrier as defined in the claims. The term "encapsulated" means to surround particles or droplets of the colorant with a protective barrier coating to provide microcapsules ranging in size from *e.g.,* about 10 µm to about 1000 µm, more typically about 100 µm to about 500 µm or even more typically about 100 µm to about 300 µm. The term "microcapsule" refers to a microparticle including a polymeric PLA shell serving as a wall-forming material and an encapsulated material, i.e. a colorant, located within the core of the microcapsule. As used herein, "microcapsules" also encompass homogeneous granules of the colorant dispersed in a PLA polymer lacking a distinctive external wall phase. Microcapsules may be any shape, *e.g.* spherical, other geometric shaped or irregularly shaped.

As described herein, the present encapsulated colorants dispersed within the present stable whitening dentifrice composition demonstrate minimal migration into a vehicle of the stable whitening dentifrice composition containing, for example, a whitening agent. Accordingly, the colorant minimally reacts with the other ingredients of the composition (e.g. the whitening agent), and the integrity of the color of the colorant is maintained. For example, the colorant does not fade or change color as may occur upon contact with a component in the vehicle, *e.g.,* the whitening agent. Further, the colorant is able to be dispersed as discrete particles, such as speckles or other patterns within the vehicle of the stable whitening dentifrice composition without bleeding or leaching into the vehicle.

### Protective barrier

In some embodiments, the protective barrier is a material having a melting point between about 80°C to about 180°C, more typically about 130 °C to about 150 °C, even more typically ranging from about 140°C to about 150°C, is able to form fine droplets when sprayed from a spray nozzle, is a solid at room temperature and liquefies without destruction upon heating. In some embodiments, the protective barrier is hydrophobic. The protective barrier is stable when in contact with whitening agents, for example, hydrogen peroxide, water or other ingredients, such as flavors, glycerin, sorbitol, surfactants and other materials, which are standardly present in dentifrices. In some embodiments, the material is one which can be dispersed in suitable solutes or creams, *etc.,* having been made into, for example, a microcapsule or microsphere.

The material used for the protective barrier is a biodegradable PLA polymer as defined in the claims.

The polymer for use with the stable whitening dentifrice composition of the present invention is polylactic acid (PLA)/poly(lactide).

According to the invention, biodegradable PLA polymers as defined in the claims are used as the protective barrier material. The term "biodegradable" as used herein refers to polymers containing chemical linkages that can be broken down by hydrolysis, enzymes and/or bacteria to form a lower molecular weight species. The biodegradable PLA polymers described in the present disclosure encompass those that meet the biodegradability criteria specified in ASTM 6400. More particularly, the ASTM 6400 criteria are designed to test the degree and rate of aerobic biodegradation of plastic materials on exposure to a controlled composting environment under laboratory conditions. The test is intended to mimic recognized composting conditions in municipal and industrial aerobic composting facilities. The parameters used are those required to determine if the plastics and products made from the plastics will compost satisfactorily, including biodegrading at a rate comparable to known compostable materials. Further, the properties in the ASTM 6400 specification are required to assure that the degradation of these materials will not diminish the value or utility of the compost resulting from the composting process. For example, ASTM 6400 criteria state that satisfactory disintegration of a test material is indicated when after twelve weeks in a controlled composting environment, no more than 10% of the test material's original dry weight remains after sieving on a 2.0-mm sieve. Further, at least 60% of the organic carbon must be converted to carbon dioxide by the end of the test period, when compared to a positive control such as cellulose. In addition, no adverse effects on the quality of the compost should be observed. For example, the germination rate and the plant biomass of the sample composts should be no less than 90 % to that of corresponding control composts for two different plant species. Further, heavy metals must meet acceptable levels as indicated by the ASTM 6400 guidelines.

The term "polylactic acid," includes any one or more of four morphologically distinct polylactic acid polymers: D-polylactic acid, L-polylactic acid, D, L-polylactic acid, and meso-polylactic acid. "D-polylactic acid" and "L-polylactic acid" are dextro-polylactic acid and levo-polylactic acid, respectively, and both of them are optically active polymers that rotate a light vector when transmitted through the polymer. "D, L-polylactic acid" is a racemic polymer, *i.e.,* a copolymer of D-polylactic acid and L-polylactic acid having a well-defined conformation of D- and L-polylactic acid units. "Meso-polylactic" is a random copolymer of D-polylactic and L-polylactic.

In some embodiments, the polylactide polymer has a weight average molecular weight (Mw) ranging from about 100,000 to about 500,000 daltons. In other embodiments, the weight average ranges from 100,000 to 300,000 daltons, and in yet other embodiments ranges from 100,000 to about 250,000 daltons.

Methods for preparing polylactic acid polymers are well known in the art. For example, polylactic acid polymers may be prepared by polycondensation (including solution polycondensation and melt polycondensation) of for example D- or L-lactic acid or combinations thereof as described herein, more typically, from ring opening polymerization of lactide, a cyclic dimer of lactic acid. Chemical modification of polylactic acid polymers may be performed by copolymerizing lactic acid with other monomers or by crosslinking or adding organic or inorganic filler/fiber materials to modify physical characteristics and/or reduce overall material cost.

Non-limiting examples of organic fillers include wood flour, seeds, polymeric particles, ungelatinized starch granules, cork, gelatins, saw dust, milled polymeric materials, agar-based materials, and the like.

Examples of inorganic fillers include calcium carbonate, titanium dioxide, silica, talc, mica, sand, gravel, crushed rock, bauxite, granite, limestone, sandstone, glass beads, aerogels, xerogels, clay, alumina, kaolin, microspheres, hollow glass spheres, porous ceramic spheres, gypsum dihydrate, insoluble salts, magnesium carbonate, calcium hydroxide, calcium aluminate, magnesium carbonate, ceramic materials, pozzolanic materials, salts, zirconium compounds, xonotlite (a crystalline calcium silicate gel), lightweight expanded clays, perlite, vermiculite, hydrated or unhydrated hydraulic cement particles, pumice, zeolites, exfoliated rock, ores, minerals, and the like. In some embodiments, 10 wt% to 30 wt%, such as 15-30%, such as 15% to 25% of organic fillers, inorganic fillers or fibers are used in the biodegradable PLA polymers of the present disclosure.

Non-limiting examples of fibers that may be incorporated into the biodegradable compositions include naturally occurring organic fibers, such as cellulosic fibers extracted from wood, plant leaves, and plant stems. These organic fibers can be derived from cotton, wood fibers (both hardwood or softwood fibers, examples of which include southern hardwood and southern pine), flax, abaca, sisal, ramie, hemp, and bagasse. In addition, inorganic fibers made from glass, graphite, silica, ceramic, rock wool, or metal materials may also be used.

Suitable commercially available polylactic acid polymers may be obtained from NatureWorks™ from Cargill Dow, USA, LACEA from Mitsui Chemicals, Japan, and Lacty from Shimadzu Seisakusho, Japan for example. In certain embodiments, polylactic acid polymers include PLA NatureWorks™ 5729B, 2002D, and 5040D available from Cargill Dow.

### Colorants

As noted above, the protective barrier encapsulates a colorant. As used herein, the term "colorant" refers to a substance in the form of a dry powder or liquid that imparts color to another substance. Generally, colorants include dyes, lakes, pigments or combinations thereof. In some embodiments, the colorant is a dye, lake or a combination of one or more dyes and/or one or more lakes.

As used herein, the term "dye" refers to an organic species, which is essentially water soluble in an aqueous medium in which the dye remains chemically stable. "Lakes" are manufactured by attaching dye molecules to insoluble reactive or adsorptive substratum, such as aluminum hydroxide particles, thereby rendering them water-insoluble.

A "pigment" is a synthetic or natural water insoluble substance, which imparts color to another substance.

The dyes used with the stable whitening dentifrice composition of the present disclosure are generally food color additives presently certified under the Food Drug & Cosmetic Act for use in food and ingested drugs, including dyes such as FD&C Red No. 3 (sodium salt of tetraiodofluorescein), FD&C Yellow No. 5 (sodium salt of 4-p-sulfophenylazo-l-p-sulfophenyl-5-hydroxypyrazole-3 carboxylic acid), FD&C Yellow No. 6 (sodium salt of p-sulfophenylazo-B-naphtol-6-monosulfonate), FD&C Green No. 3 (disodium salt of 4-{[4-(N-ethyl-p-sulfobenzylamino)-phenyl]-(4-hydroxy-2-sulfoniumphenyl)-m ethylene}-[1-N-ethyl-N-p-sulfobenzyl)-.DELTA.-3,5-cyclohexadienimine], FD&C Blue No. 1 (disodium salt of dibenzyldiethyl-diaminotriphenylcarbinol trisulfonic acid anhydride), FD&C Blue No. 2 (sodium salt of disulfonic acid of indigotin) D&C Green No. 5, D&C Orange No. 5, D&C Red No. 21, D&C Red No. 22, D&C Red No. 27, D&C Red No. 28, D&C Red No. 30, D&C Red No. 40, D&C Yellow No. 10 and mixtures thereof in various proportions.

Lakes which may be used with the stable whitening dentifrice composition of the present disclosure include but are not limited to FD&C Blue No. 1 Lake, FD&C Blue No. 2 Lake, FD&C Green No. 3 Lake, FD&C Red No. 3 Lake, FD&C Red No. 40 Lake, FD&C Yellow No. 5 Lake, FD& C Yellow No. 6 Lake. In certain embodiments the colorant is selected from FD&C Blue No. 1 Lake, FD&C Yellow No. 5 Lake, and FD&C Red No. 40 Lake.

In some embodiments, pigments are optionally added to the dye and/or lake colorants. Pigments include non-toxic, water insoluble inorganic pigments such as titanium dioxide and chromium oxide greens, ultramarine blues and pinks and ferric oxides, e.g., annatto extract, chlorophyllin-copper complex, canthaxanthin, β-carotene, synthetic iron oxide, TiO₂ and mixtures thereof.

Suitable dyes and lakes, their structures, and properties for use herein are well known to those skilled in the art, *see, for example,* Kirk-Othmer Encyclopedia of Chemical Technology, 3rd Edition, Volume 6, pg. 561-596.

In some embodiments, the whitening compositions are stable. As used herein "stable" means that migration of the colorant into the vehicle of the present whitening dentifrice composition is suppressed, mitigated or prevented by the protective barrier for a period of time, such that a reaction of the colorant with the other ingredients of the whitening composition resulting in a change in the colorant, for example fading of the colorant, is not visually observed. "Stable" also refers to the lack or suppression of ability or the lack or suppression of propensity of the encapsulated colorant to leach or bleed or migrate into the vehicle of the present stable whitening dentifrice composition, such that the integrity of discrete particles or patterns of the encapsulated colorant in the vehicle may be maintained when visually observed. As used herein "not visually observed" or "not visually observable" means that the colorant encapsulated in the protective coating is not visualized or observable as bleeding or leaching into the vehicle or that fading or a color change of the colorant is not visualized or observable when viewed with the naked eye.

In various embodiments, migration of colorant encapsulated in the protective barrier is suppressed and not visually observed at temperatures ranging from about 20°C to about 50°C, for example from about 20°C to about 40°C or about 30°C or about 40°C.

In some embodiments, migration of the colorant into the vehicle is suppressed and not visually observable for a duration of time or a period of time, such as about one day, about one week, about one month, about two months, about three months, about four months, about six months, about one year or about two years or more.

The term "stable" as used herein also encompasses migration of a specified amount of the colorant into the present vehicle of the whitening dentifrice composition of the instant disclosure where the amount is about 0%, less than about 1%, less than about 2%, less than about 3%, less than about 4%, less than about 5%, less than about 10% or less than about 20% at temperatures ranging from about 20°C to about 50°C, for example from about 20°C to about 40°C or about 30°C or about 40°C, for a specified duration of time, where the duration is about one day, about one week, about one month, about two months, about three months, about four months, about six months, about one year or about two years or more.

Colorants that are encapsulated in a protective coating and are suitable for use with the present stable whitening dentifrice composition are commercially available. For example, FD & C Blue No. 1 Lake surrounded by a polylactic acid protective coating (EcoBlue 50BL1) may be purchased from Micro Powders, Inc., Tarrytown, NY.

In some embodiments, colorants encapsulated in a protective barrier may be prepared using methods well known in the art including but not limited to spray drying, interfacial polymerization, hot melt encapsulation, phase separation encapsulation (solvent removal and solvent evaporation), spontaneous emulsion and coacervation, *see, for example,* Kirk-Othmer Encyclopedia of Chemical Technology, Third Edition, Vol. 125 Pgs. 470-493 (1981).

An example of a method by which colorants, such as lakes or dyes of the present disclosure, may be encapsulated in polylactic acid, is by dispersing the desired amount of colorant in the polymer that has been thermally softened to form a liquid composition. The ratio of lake or dye to polylactic acid, is between about 0.5% to about 9%, more typically about 2% to about 6%.

The desired amount of colorant, in some embodiments, is the amount of colorant which results in a final concentration of up to 20% colorant in the present dentifrice compositions, for example, about 0.1% to about 12%, about 0.1% to about 10%, about 3% to about 10%, about 0.1 to about 5%, about 0.1% to about 2% or about 0.1% to about 1% by weight.

The liquid polymer dispersion may be agitated so that the liquid polymer deposits on each entity of the dye or lake material forms liquid polymer walled droplets. The dispersion is then cooled and milled to provide solid particles in which the dye or lake is encapsulated. The particle size distribution of the polymer particles can vary from about 10 µm to about 1000 µm.

### Whitening Agent

According to the invention, the whitening agent is an oxidizing agent, a reducing agent or combinations thereof. In its broadest sense, "oxidizing agent" is intended to include those compounds which can accept an electron from another molecule in the environment of the oral cavity without having a deleterious or unacceptably harmful affect on the oral cavity in normal and accepted use.

Oxidizing agents suitable for use with the present stable whitening dentifrice composition include peroxides, metal chlorites and persulfate. Exemplary peroxides include hydroperoxides, hydrogen peroxide, peroxides of alkali and alkaline earth metals, organic peroxy compounds, peroxy acids, pharmaceutically-acceptable salts thereof, and mixtures thereof.

Peroxides of alkali and alkaline earth metals include lithium peroxide, potassium peroxide, sodium peroxide, magnesium peroxide, calcium peroxide, barium peroxide, and mixtures thereof. Organic peroxy compounds include urea peroxide, glyceryl hydrogen peroxide, alkyl hydrogen peroxides, dialkyl peroxides, alkyl peroxy acids, peroxy esters, diacyl peroxides, benzoyl peroxide, and monoperoxyphthalate, and mixtures thereof. Peroxy acids and their salts include organic peroxy acids such as alkyl peroxy acids, and monoperoxyphthalate and mixtures thereof, as well as inorganic peroxy acid salts such as perborate salts of alkali and alkaline earth metals such as lithium, potassium, sodium, magnesium, calcium and barium, and mixtures thereof.

In some embodiments, the oxidizing agent comprises hydrogen peroxide. In some embodiments, the oxidizing agent comprises from 0.1 % to 50% w/w, such as from 0.1 % to 40% w/w, and more typically from 0.1% to 30% w/w, of the stable whitening dentifrice composition. In other embodiments, the oxidizing agent consists essentially of hydrogen peroxide. In some embodiments, the hydrogen peroxide comprises from 0.1 % to 3% w/w, such as from 0.1 % to 2% w/w, and more typically about 1% to 4% w/w of the stable whitening dentifrice composition.

In other embodiments, the whitening agent is a reducing agent. In its broadest sense, this term is intended to include those compounds which can donate an electron to another molecule in the environment of the oral cavity without having a deleterious or unacceptably harmful affect on the oral cavity in normal and accepted use. Synonyms for this term are preservatives or anti-oxidizing agents. There are numerous compounds which have been proven to be useful as reducing agents. A list of such compounds currently recognized for this purpose can be found in reference manuals and compendia covering pharmaceutical and oral care products. For example, *see* Martindale the Extra Pharmacopoeia, The Pharmaceutical Press, (London), Thirtieth Ed. James E. F. Reynolds Ed. (1993) beginning at page 1132-1139 (Martindale). In so far as they meet the standards of oral acceptability, any compounds known in the art have the potential to be used in the stable whitening dentifrice composition of the present disclosure, e.g., vitamin C and its esters, vitamin E, the benzoates and hydroxybenzoates, butylated hydroxytoluene (BHT), butylated hydroxyanisole (BHA) and other reducing phenols, derivatives of dihydroxyquinoline, derivatives of polymerized 2,2,4- trimethyl-1,2-dihydroquinoline and alkyl gallate such as dodecyl gallate, ethyl gallate, octyl gallate and propyl gallate. In some embodiments, vitamin C, vitamin E, BHA, BHT, propyl gallate and combinations thereof are used.

In some embodiments, a solid whitening agent, such as sodium perborate, urea peroxide, calcium chlorite, barium chlorite, magnesium chlorite, lithium chlorite, sodium chlorite, potassium chlorite or mixtures thereof are used in the stable whitening dentifrice composition described herein.

In some embodiments, the whitening agent is bound, for example, to a polymer, such as PVP (poly(N-vinylpyrrolidone). Suitable PVP complexes are disclosed, for example, in U.S. Patent No. 5,122,370.

### Vehicle

The whitening agent as described herein is combined with an orally acceptable anhydrous vehicle including the colorant encapsulated in a protective barrier to form a dentifrice. Such dentifrices may include a dental tablet, toothpaste (dental cream), tooth powders, or gel, or any other form known to one of skill in the art.

As used herein, an "orally acceptable vehicle" refers to a material or combination of materials that are safe for use in the compositions of the present disclosure, commensurate with a reasonable benefit/risk ratio, with which the whitening agent, and other desired active ingredients may be associated while retaining significant efficacy. In some embodiments, the combination of ingredients is acidic to maintain stability of the whitening agent.

According to the invention, the vehicle is anhydrous and may include any known ingredients or additives. Suitable low water content humectants/vehicles include but are not limited to polyethylene glycol, such as PEG400, PEG600, PEG/PPG copolymers, such as PEG/PPG 38/8 copolymer, PEG/PPG-1 16/66 copolymer sold as PLURACARE® L4370 and PLURACARE® L1220 from BASF, Wyandotte, Michigan, respectively. In some embodiments, the humectant/vehicle is present in a total amount of from 2% to 55% w/w and typically from 2% to 35% w/w. Propylene glycol or glycerin may also be present in an amount from 0% to 15% w/w.

In some embodiments, the viscosity of the dentifrice is between 10,000 cps and 1,000,000cps. In other embodiments, the viscosity of the dentifrice is between 30,000 cps and 300,000 cps. In some embodiments, the viscosity of the dentifrice is between 60,000 cps to 700,000 cps.

In some embodiments, the vehicle may comprise surfactants. In some embodiments, surfactants enhance stability of the formulation, help clean the oral cavity surfaces through detergency, and provide foam upon agitation, e.g., during brushing with a dentifrice composition of the disclosure. Surface active agents generally achieve increased prophylactic action, by thoroughly dispersing the whitening agent throughout the oral cavity. In various embodiments, suitable surface active agents may function as a surface active agent, emulsifier, and/or foam modulator.

Any orally acceptable surfactant, most of which are anionic, nonionic or amphoteric, can be used. Suitable anionic surfactants include without limitation watersoluble salts of C₈₋₂₀ alkyl sulfates, sulfonated monoglycerides of C₈₋₂₀ fatty acids, sarcosinates, taurates and the like. Illustrative examples of these and other classes include sodium lauryl sulfate, sodium cocoyl monoglyceride sulfonate, sodium lauryl sarcosinate, sodium lauryl isoethionate, sodium laureth carboxylate and sodium dodecyl benzenesulfonate. Suitable nonionic surfactants include without limitation poloxamers, polyoxyethylene sorbitan esters, fatty alcohol ethoxylates, alkylphenol ethoxylates, tertiary amine oxides, tertiary phosphine oxides, dialkyl sulfoxides and the like. Suitable amphoteric surfactants include without limitation derivatives of C₈₋₂₀aliphatic secondary and tertiary amines having an anionic group such as carboxylate, sulfate, sulfonate, phosphate or phosphonate. A suitable example is cocoamidopropyl betaine.

In some embodiments, one or more surfactants may be present in a total amount of from about 1.8% to about 2% w/w. In some embodiments, one or more surfactants may be present in a total amount of from about 1.9% to about 2% w/w. In some embodiments, one or more surfactants may be present in a total amount of about 2% w/w.

In some embodiments, the composition optionally comprises a thickening agent. Any orally acceptable thickening agent can be used, including without limitation carbomers, also known as carboxyvinyl polymers, carrageenans, also known as Irish moss and more particularly -carrageenan (iota-carrageenan), high molecular weight polyethylene glycols (such as CARBOWAX, available from The Dow Chemical Company), cellulosic polymers such as hydroxyethylcellulose, carboxymethylcellulose (CMC) and salts thereof, *e.g.,* CMC sodium, natural gums such as karaya, xanthan, gum arabic and tragacanth, colloidal magnesium aluminum silicate, and colloidal and/or fumed silica and mixtures of the same. In some embodiments, the one or more optional thickening agents are present in a total amount of about 0.1 % to about 90% w/w. In some embodiments, the one or more optional thickening agents are present in a total amount of about 1% to about 50% w/w. In some embodiments, the one or more optional thickening agents are present in a total amount of about 5% to about 35% w/w.

Useful flavoring agents include any material or mixture of materials operable to enhance the taste of the composition. Any orally acceptable natural or synthetic flavoring agent can be used, such as flavoring oils, flavoring aldehydes, esters, alcohols, similar materials, and combinations thereof. Flavoring agents include vanillin, sage, marjoram, parsley oil, spearmint oil, cinnamon oil, oil of wintergreen (methylsalicylate), peppermint oil, clove oil, bay oil, anise oil, eucalyptus oil, citrus oils, fruit oils and essences including those derived from lemon, orange, lime, grapefruit, apricot, banana, grape, apple, strawberry, cherry, pineapple, etc., bean- and nut-derived flavors such as coffee, cocoa, cola, peanut, almond, etc., adsorbed and encapsulated flavorants, and mixtures thereof. Also encompassed within flavoring agents herein are ingredients that provide fragrance and/or other sensory effect in the mouth, including cooling or warming effects. Such ingredients include menthol, menthyl acetate, menthyl lactate, camphor, eucalyptus oil, eucalyptol, anethole, eugenol, cassia, oxanone, x-irisone, propenyl guaiethol, thymol, linalool, benzaldehyde, cinnamaldehyde, N-ethyl-p-menthan-3-carboxamine, N,2,3-trimethyl-2-isopropylbutanamide, 3-1-menthoxypropane-1,2-diol, cinnamaldehyde glycerol acetal (CGA), methone glycerol acetal (MGA) and mixtures thereof.

In some embodiments, one or more flavoring agents are optionally present in a total amount of about 0.01% to about 5% w/w. In some embodiments, one or more flavoring agents are optionally present in a total amount of about 0.05% to about 2% w/w. In some embodiments, one or more flavoring agents are optionally present in a total amount of about 0.1% to about 2.5% w/w. In some embodiments, one or more flavoring agents are optionally present in a total amount from about 0.1 % to about 0.5% w/w. In some embodiments, one or more flavoring agents are optionally present in the total amount of about 2.25% w/w.

Sweeteners among those useful herein include orally acceptable natural or artificial, nutritive or non-nutritive sweeteners. Such sweeteners include dextrose, polydextrose, sucrose, maltose, dextrin, dried invert sugar, mannose, xylose, ribose, fructose, levulose, galactose, corn syrup (including high fructose corn syrup and corn syrup solids), partially hydrolyzed starch, hydrogenated starch hydrolysate, sorbitol, mannitol, xylitol, maltitol, isomalt, aspartame, neotame, saccharin and salts thereof, sucralose, dipeptide-based intense sweeteners, cyclamates, dihydrochalcones and mixtures thereof. Some embodiments optionally comprise one or more sweeteners. In some embodiments, the one or more optional sweeteners are present in a total amount from about 0.005% to about 5% w/w. In some embodiments, the one or more optional sweeteners are present in a total amount from about 0.01 to about 1 % w/w.

### Other Active Ingredients

The compositions of the present disclosure optionally comprise one or more further active material(s), which is operable for the prevention or treatment of a condition or disorder of hard or soft tissue of the oral cavity, the prevention or treatment of a physiological disorder or condition, or to provide a cosmetic benefit.

Some embodiments of the present disclosure comprise a dental abrasive or combination of dental abrasive agents. As used herein, the term "abrasive" or "abrasive agent" also includes materials commonly referred to as "polishing agents." Any orally acceptable abrasive can be used, but typically, type, fineness (particle size) and amount of abrasive should be selected so that tooth enamel is not excessively abraded in normal use of the composition. Suitable abrasives include without limitation silica (in the form of silica gel, hydrated silica or precipitated silica), alumina, insoluble phosphates, calcium carbonate, resinous abrasives such as urea-formaldehyde condensation products and the like.

Among insoluble phosphates useful as abrasives are orthophosphates, polymetaphosphates and pyrophosphates. Illustrative examples are dicalcium orthophosphate dihydrate, calcium pyrophosphate, n-calcium pyrophosphate, tricalcium phosphate, calcium polymetaphosphate and insoluble sodium polymetaphosphate.

Average particle size of an abrasive, if present, is generally about 0.1 to about 30 µm for example about 1 to about 20 µm or about 5 to about 15 µm. In some embodiments, one or more abrasives are present in an amount of about 0.1 % to about 40% w/w. In some embodiments, the abrasive is calcium pyrophosphate. In some embodiments, the calcium pyrophosphate is present in an amount from about 5% to about 50% w/w.

In various embodiments of the present disclosure, the stable whitening dentifrice composition comprises an anticalculus agent. Suitable anticalculus agents include without limitation phosphates and polyphosphates (for example pyrophosphates), polyaminopropanesulfonic acid (AMPS), hexametaphosphate salts, zinc citrate trihydrate, polypeptides, polyolefin sulfonates, polyolefin phosphates, diphosphonates. In some embodiments, the anticalculus agent is present in an amount of about 0.1% to about 30% w/w. In some embodiments, the stable whitening dentifrice composition comprises a mixture of anticalculus agents. In some embodiments, tetrasodium pyrophosphate (TSPP) and sodium tripolyphosphate (STPP) are used as the anticalculus agents. In some embodiments, the anticalculus agent comprises about 1% to about 2% w/w TSPP, and about 0% to about 7% w/w STPP.

Another desirable component of the present compositions is a synthetic anionic polymeric polycarboxylate (SAPP), which acts as a stabilizer for the polyphosphate anti-tartar agent and may help to block access of painful or pain-causing materials, such as sugars, to the tooth nerves.

In some embodiments, the stable whitening dentifrice composition optionally comprises a source of fluoride ions. In some embodiments, the source of fluoride ions is selected from: fluoride, monofluorophosphate (MFP), and fluorosilicate salts. In some embodiments, one or more fluoride ion-releasing compounds are optionally present in an amount providing a total of 100 to 20,000 ppm, 200 to 5,000 ppm, or 500 to 2,500 ppm, fluoride ions.

The compositions also may include a stannous ion or a stannous ion source to mitigate calcium loss. Suitable stannous ion sources include without limitation stannous fluoride, other stannous halides such as stannous chloride dihydrate, stannous pyrophosphate, organic stannous carboxylate salts such as stannous formate, acetate, gluconate, lactate, tartrate, oxalate, malonate and citrate, stannous ethylene glyoxide and the like. One or more stannous ion sources are optionally and illustratively present in a total amount of about 0.01% to about 10%, for example about 0.1% to about 7% or about 1% to about 5%.

The compositions of the present disclosure optionally comprise an antimicrobial (e.g., antibacterial) agent. An illustrative list of useful antibacterial agents is provided in U.S. Patent No. 5,776,435 to Gaffar et al.*.* One or more antimicrobial agents are optionally present in an antimicrobial effective total amount, typically about 0.05% to about 10%, for example about 0.1% to about 3%.

### Methods

In various embodiments, the present disclosure provides methods of preparing a whitening dentifrice including mixing a whitening agent into an orally acceptable vehicle to form a mixture (a) and mixing a colorant encapsulated in a protective barrier into the mixture formed in (a) to form the whitening dentifrice.

For example, in some embodiments desired humectants may be combined and mixed to form a homogenous mixture. Sweeteners and other active ingredients, such as anticalculus agents, may then be added and mixed into the homogenous humectant mixture. Bound whitening agents and optionally abrasives may be added to the foregoing mixture followed by mixing in flavors and surfactants. The colorants encapsulated by the present protective barrier may then be dispersed throughout the vehicle containing the whitening agents and other optional active ingredients.

The encapsulated colorants may vary by size and/or color and may be dispersed throughout the vehicle containing the whitening agent, such that the stable whitening dentifrice composition appears to contain speckles, speckled stripes, wavy stripes, straight stripes, spots, dots or other pattern formed by the encapsulated colorants. In other embodiments, the encapsulated colorants may be formulated and added in sufficient quantity such that the entire present whitening dentifrice composition visually appears to be one solid color.

Methods are also provided herein to whiten an oral surface in a human or animal subject comprising contacting a tooth surface with a whitening dentifrice composition of the present disclosure, which includes at least one encapsulated colorant that imparts a desirable appearance to the stable whitening dentifrice composition. As used herein "animal subject" includes non-human mammals such as canines, felines, and horses. The stable whitening dentifrice composition is contacted with an oral surface of the mammalian subject to thereby whiten teeth in a highly efficacious manner.

In various embodiments, the stable whitening dentifrice composition prepared in accordance with the present disclosure may be applied regularly to an oral surface, for example on a daily basis, at least one time daily for multiple days, or alternately every second or third day. In some embodiments, the stable whitening dentifrice composition is applied to the oral surfaces from 1 to 3 times daily, for at least 2 weeks up to 8 weeks, from four months to three years, or more up to lifetime.

The examples and other embodiments described herein are exemplary and not intended to be limiting in describing the full scope of compositions and methods of this disclosure.

### EXAMPLES

### Example 1

A whitening dentifrice was prepared by mixing the ingredients of Table 1, below. First, a flavor premix was prepared by adding BHT into the flavor mix until the BHT was fully dissolved. In a main vessel, propylene glycol, glycerin, PEG600 and PLURACARE® L1220 were mixed to form a homogenous solution. TSPP, SAPP, MFP, saccharin and sucralose were then added to the main vessel and mixed until homogenous. PVP and PVP bound to hydrogen peroxide, fumed silica and calcium pyrophosphate were added to the main vessel and mixed under vacuum to form a homogenous mixture. The flavor premix and sodium lauryl sulfate were also subsequently added to the main vessel and mixed under vacuum until a homogenous mixture was formed.

FD & C Blue No. 1 Lake encapsulated in polylactic acid was purchased from Micro Powders, Inc., Tarrytown, NY. The encapsulated colorant was dispersed throughout the homogenous mixture obtained as described above to form a blue speckled pattern in the substantially white mixture, which may be desirable or preferable to consumers.

The dentifrice was stored for three months at approximately 40° C. As is evident from FIG. 1A, migration of the colorant into the vehicle was not visually observed. Accordingly, the blue color of the FD & C Blue No. 1 Lake and the speckled pattern was maintained throughout the storage period.

**Table 1. Peroxide toothpaste with FD & C Blue No. 1 Lake speckles**

| **Ingredient** | **Percentage** |
|---|---|
| Propylene Glycol | 33.41 |
| Glycerin | 10 |
| Calcium pyrophosphate | 15 |
| PLURACARE® L1220 | 10 |
| PEG 600 | 10 |
| PVP/Peroxide complex | 5.5 |
| PVP | 3.375 |
| Fumed silica | 3.375 |
| Flavor | 2.25 |
| TSPP | 2 |
| Sodium lauryl sulfate | 2 |
| SAPP | 0.9 |
| MFP | 0.76 |
| Sodium Saccharin | 0.6 |
| Sucralose | 0.05 |
| BHT | 0.03 |
| Polylactic Acid Speckle with FD & C Blue No. 1 Lake | 0.75 |
| **Total** | **100** |

### Comparative Example 1

A comparative, whitening dentifrice was prepared by mixing the ingredients of Table 1, except that non-encapsulated FD&C Blue No. 1 Lake was added to the dentifrice. After aging the dentifrice for only two weeks at approximately 40° C, the colorant was no longer blue, but a faded green, resulting from contact with the whitening agent as shown in FIG. 1B.

## Claims

1. A stable whitening dentifrice composition comprising:
an orally acceptable vehicle comprising a whitening agent, and
a colorant encapsulated by a protective barrier,
wherein the whitening agent is selected from the group consisting of an oxidizing agent, a reducing agent and a combination thereof,
wherein the protective barrier suppresses migration of the colorant into the orally acceptable vehicle for a duration of time of at least about 1 day;
wherein the orally acceptable vehicle is anhydrous,
wherein said protective barrier is a biodegradable polymer, which is polylactic acid (PLA).

2. The stable whitening dentifrice composition of claim 1, wherein the oxidizing agent is selected from the group consisting of a peroxide, a metal chlorite, a persulfate and combinations thereof.

3. The stable whitening dentifrice composition of claim 2, wherein the peroxide is selected from the group consisting of lithium peroxide, potassium peroxide, sodium peroxide, magnesium peroxide, calcium peroxide, barium peroxide, hydrogen peroxide, benzoyl peroxide, urea peroxide, and mixtures thereof.

4. The stable whitening dentifrice composition of claim 1, wherein the whitening agent is hydrogen peroxide, optionally in an amount of about 1-4% w/w.

5. The stable whitening dentifrice composition of claim 1, wherein the reducing agent is selected from the group consisting of vitamin C, vitamin E, benzoate, butylated hydroxytoluene (BHT), butylated hydroxyanisole (BHA) propyl gallate and combinations thereof.

6. The stable whitening dentifrice composition of claim 1, wherein the orally acceptable vehicle comprises polyethylene glycol, ethylene oxide, propylene oxide and combinations thereof.

7. The stable whitening dentifrice composition of claim 1, wherein the colorant is a lake, a dye or a combination thereof.

8. The stable whitening dentifrice composition of claim 1, wherein the colorant is a lake, optionally wherein said lake is selected from the group consisting of FD & C Blue No. 1 Lake, FD & C Blue No. 2 Lake, FD & C Red No. 3 Lake, FD & C Yellow No. 5 Lake, FD & C Yellow No. 6 Lake and combinations thereof.

9. The stable whitening dentifrice composition of claim 1, wherein the lake is FD & C Blue No. 1 Lake.

10. The stable whitening dentifrice composition of claim 7, wherein the colorant is
a dye, optionally wherein said dye is selected from the group consisting of FD&C Red No. 3, Food Red 17, Food Yellow 13, FD&C Yellow No. 5, FD&C Yellow No. 6, FD&C Green No. 3, FD&C Blue No. 1, FD&C Blue No. 2 and combinations thereof.

11. The stable whitening dentifrice composition of claim 1, wherein the colorant encapsulated by a protective barrier causes the composition to have a speckled appearance.

12. The stable whitening dentifrice composition of claim 1, wherein the duration of time is at least about three months or is at least about two years.

## Patentansprüche

1. Stabile aufhellende Zahnreinigungsmittel-Zusammensetzung, umfassend ein oral verträgliches Vehikel, das ein Aufhellungsmittel umfasst, und
einen Farbstoff, der durch eine schützende Barriere eingekapselt ist,
wobei das Aufhellungsmittel ausgewählt ist aus der Gruppe bestehend aus einem Oxidationsmittel, einem Reduktionsmittel und einer Kombination davon,
wobei die schützende Barriere die Migration des Farbstoffs in das oral verträgliche Vehikel für eine Zeitdauer von mindestens etwa 1 Tag unterdrückt;
wobei das oral verträgliche Vehikel wasserfrei ist,
wobei die schützende Barriere ein biologisch abbaubares Polymer ist, das Polymilchsäure (PLA) ist

2. Stabile aufhellende Zahnreinigungsmittel-Zusammensetzung nach Anspruch 1, wobei das Oxidationsmittel ausgewählt ist aus der Gruppe bestehend aus einem Peroxid, einem Metallchlorit, einem Persulfat und Kombinationen davon.

3. Stabile aufhellende Zahnreinigungsmittel-Zusammensetzung nach Anspruch 2, wobei das Peroxid ausgewählt ist aus der Gruppe bestehend aus Lithiumperoxid, Kaliumperoxid, Natriumperoxid, Magnesiumperoxid, Calciumperoxid, Bariumperoxid, Wasserstoffperoxid, Benzoylperoxid, Harnstoffperoxid und Mischungen davon.

4. Stabile aufhellende Zahnreinigungsmittel-Zusammensetzung nach Anspruch 1, wobei das Aufhellungsmittel Wasserstoffperoxid ist, gegebenenfalls in einer Menge von etwa 1-4 % Gew/Gew.

5. Stabile aufhellende Zahnreinigungsmittel-Zusammensetzung nach Anspruch 1, wobei das Reduktionsmittel ausgewählt ist aus der Gruppe bestehend aus Vitamin C, Vitamin E, Benzoat, butyliertem Hydroxytoluol (BHT), butyliertem Hydroxyanisol (BHA), Propylgallat und Kombinationen davon.

6. Stabile aufhellende Zahnreinigungsmittel-Zusammensetzung nach Anspruch 1, wobei das oral verträgliche Vehikel Polyethylenglykol, Ethylenoxid, Propylenoxid und Kombinationen davon umfasst.

7. Stabile aufhellende Zahnreinigungsmittel-Zusammensetzung nach Anspruch 1, wobei der Farbstoff ein Lack, ein Färbemittel oder eine Kombination davon ist.

8. Stabile aufhellende Zahnreinigungsmittel-Zusammensetzung nach Anspruch 1, wobei das Färbemittel ein Lack ist, wobei der Lack gegebenenfalls aus der Gruppe ausgewählt ist, die aus FD & C Blau Nr. 1 Lack, FD & C Blau Nr. 2 Lack, FD & C Rot Nr. 3 Lack, FD & C Gelb Nr. 5 Lack, FD & C Gelb Nr. 6 Lack und Kombinationen davon besteht.

9. Stabile aufhellende Zahnreinigungsmittel-Zusammensetzung nach Anspruch 1, wobei der Lack FD & C Blau Nr. 1 Lack ist.

10. Stabile aufhellende Zahnreinigungsmittel-Zusammensetzung nach Anspruch 7, wobei das Färbemittel ein Farbstoff ist, wobei der Farbstoff gegebenenfalls ausgewählt ist aus der Gruppe bestehend aus FD&C Rot Nr. 3, Lebensmittelrot 17, Lebensmittelgelb 13, FD&C Gelb Nr. 5, FD&C Gelb Nr. 6, FD&C Grün Nr. 3, FD&C Blau Nr. 1, FD&C Blau Nr. 2 und Kombinationen davon.

11. Stabile aufhellende Zahnreinigungsmittel-Zusammensetzung nach Anspruch 1, wobei das Färbemittel, das durch eine schützende Barriere eingekapselt ist, bewirkt, dass die Zusammensetzung ein gesprenkeltes Aussehen aufweist.

12. Stabile aufhellende Zahnreinigungsmittel-Zusammensetzung nach Anspruch 1, wobei die Zeitdauer mindestens etwa drei Monate oder mindestens etwa zwei Jahre beträgt.

## Revendications

1. Composition de dentifrice blanchissante stable comprenant :
un véhicule acceptable par voie orale comprenant un agent de blanchiment, et
un colorant encapsulé par une barrière protectrice,
dans laquelle l'agent de blanchiment est choisi dans le groupe constitué par un agent oxydant, un agent réducteur et une combinaison de ceux-ci,
dans laquelle la barrière protectrice supprime la migration du colorant dans le véhicule acceptable par voie orale pendant une durée d'au moins environ 1 jour ;
dans laquelle le véhicule acceptable par voie orale est anhydre,
dans laquelle ladite barrière protectrice est un polymère biodégradable, qui est l'acide polylactique (PLA).

2. Composition de dentifrice blanchissante stable selon la revendication 1, dans laquelle l'agent oxydant est choisi dans le groupe constitué par un peroxyde, un chlorite métallique, un persulfate et une combinaison de ceux-ci.

3. Composition de dentifrice blanchissante stable selon la revendication 2, dans laquelle le peroxyde est choisi dans le groupe constitué par le peroxyde de lithium, le peroxyde de potassium, le peroxyde de sodium, le peroxyde de magnésium, le peroxyde de calcium, le peroxyde de baryum, le peroxyde d'hydrogène, le peroxyde de benzoyle, le peroxyde d'urée et les mélanges de ceux-ci.

4. Composition de dentifrice blanchissante stable selon la revendication 1, dans laquelle l'agent de blanchiment est le peroxyde d'hydrogène, éventuellement en une quantité d'environ 1 à 4 % p/p.

5. Composition de dentifrice blanchissante stable selon la revendication 1, dans laquelle l'agent réducteur est choisi dans le groupe constitué par la vitamine C, la vitamine E, le benzoate, l'hydroxytoluène butylé (BHT), l'hydroxyanisole butylé (BHA), le gallate de propyle et les combinaisons de ceux-ci.

6. Composition de dentifrice blanchissante stable selon la revendication 1, dans laquelle le véhicule acceptable par voie orale comprend le polyéthylèneglycol, l'oxyde d'éthylène, l'oxyde de propylène et les combinaisons de ceux-ci.

7. Composition de dentifrice blanchissante stable selon la revendication 1, dans laquelle le colorant est une laque, une matière colorante ou une combinaison de celles-ci.

8. Composition de dentifrice blanchissante stable selon la revendication 1, dans laquelle le colorant est une laque, éventuellement dans laquelle ladite laque est choisie dans le groupe constitué par la FD & C Blue No. 1 Lake, la FD & C Blue No. 2 Lake, la FD & C Red No. 3 Lake, la FD & C Yellow No. 5 Lake, la FD & C Yellow No. 6 Lake et les combinaisons de celles-ci.

9. Composition de dentifrice blanchissante stable selon la revendication 1, dans laquelle la laque est la FD & C Blue No. 1 Lake.

10. Composition de dentifrice blanchissante stable selon la revendication 7, dans laquelle le colorant est une matière colorante, éventuellement dans laquelle ladite matière colorante est choisie dans le groupe constitué par FD & C Red No. 3, Food Red 17, Food Yellow 13, FD & C Yellow No. 5, FD & C Yellow No. 6, FD & C Green No. 3, FD & C Blue No. 1, FD & C Blue No. 2 et les combinaisons de ceux-ci.

11. Composition de dentifrice blanchissante stable selon la revendication 1, dans laquelle le colorant encapsulé par une barrière protectrice amène la composition à présenter un aspect moucheté.

12. Composition de dentifrice blanchissante stable selon la revendication 1, dans laquelle la durée est d'au moins environ trois mois ou est d'au moins environ deux ans.
